# EUROPEAN PATENT SPECIFICATION

(11) **EP 2 830 573 B1**
(45) Date of publication and mention of the grant of the patent: **22.04.2020**
(21) Application number: 13712791.6
(22) Date of filing: 27.03.2013
(51) Int. Cl.: A61K 8/22, A61K 8/31, A61Q 5/08, A61K 8/38

(54) **Multi-compartment device for bleaching keratin fibres**
Mehrkomponentenpackung zum Bleichen von Keratinfasern
Kit à plusieurs compartiments pour la décoloration de fibres kératiniques

(30) Priority: 30.03.2012 FR 1252901; 30.04.2012 US 201261640211 P
(43) Date of publication of application: 04.02.2015
(73) Proprietor: L'OREAL, 75008 Paris (FR)
(72) Inventor: DECONINCK, Gautier, F-59200 Tourcoing (FR)
(74) Representative: L'Oreal
(86) International application number: PCT/EP2013/056595
(87) International publication number: WO 2013/144246

(56) References cited:
- EP-A1- 0 659 400
- EP-A1- 2 417 959
- DE-C1- 19 723 538
- FR-A1- 2 438 477
- FR-A1- 2 788 975
- FR-A1- 2 958 155
- US-A1- 2002 179 109

## Description

The subject of the present invention is a multi-compartment device comprising compositions for bleaching keratin fibres, in particular the hair, with a bleaching composition resulting from the mixing of an anhydrous composition A comprising at least one peroxygenated salt, of an aqueous composition B comprising at least one basifying agent and of a composition C comprising at least one oxidizing agent, said bleaching composition comprising at least 10% by weight of fatty substance.
The bleaching of human keratin fibres, and more particularly the hair, is performed by oxidation of the "melanin" pigment resulting in the dissolution and the partial or total removal of this pigment.
To bleach the hair, use is in particular made of bleaching compositions containing a peroxygenated reagent, such as ammonium persulfates, perborates and percarbonates or alkali metal persulfates, perborates and percarbonates, which is combined, at the moment of use, with an aqueous composition comprising at least one oxidizing agent such as hydrogen peroxide, under alkaline pH conditions in the vast majority of cases. The alkaline agent most commonly used is aqueous ammonia, which makes it possible to adjust the pH of the composition to an alkaline pH to enable activation of the oxidizing agent. However, this alkaline agent also causes swelling of the keratin fibre, with opening of the scales, thereby promoting penetration of the oxidizing agent into the fibre, and therefore increases the efficiency of the reaction.

As it happens,this basifying agent is highly volatile, and this causes unpleasantness to the user on account of the strong and fairly unpleasant characteristic odour of ammonia that is given off during the process.
Moreover, the amount of ammonia given off requires the use of levels which are greater than those necessary, in order to compensate for this loss. This is not without consequence for the user, who not only remains inconvenienced by the odour but may also be confronted with greater risks of intolerance, such as, for example, irritation of the scalp reflected in particular by stinging sensations.

Furthermore, it is sought to improve the ease of mixing and application of bleaching compositions, which are generally in the form of a powder or paste, which is mixed at the moment of use with the aqueous oxidizing composition.

Thus, one of the objectives of the present invention is to propose devices containing compositions to be mixed for bleaching human keratin fibres that do not have the drawbacks of those used with the existing compositions, these drawbacks being caused by the presence of large amounts of aqueous ammonia, but that remain at least as efficient in terms of the bleaching and the uniformity of this bleaching.

Another object of the present invention is to propose devices containing compositions to be mixed for bleaching keratin fibres which are easy to mix and to apply, in particular which enable a uniform distribution of the composition on the hair.

These objectives are achieved with the present invention, the subject of which is a multi-compartment device as defined in claim 1.

In the context of the present invention, a composition is anhydrous when it has a water content of less than 1% by weight and preferably less than 0.5% by weight relative to the total weight of the composition.
For the purposes of the present invention, an aqueous composition comprises more than 5% by weight of water, preferably more than 10% by weight of water and even more advantageously more than 20% by weight of water.

In the text hereinbelow, and unless otherwise indicated, the limits of a range of values are included in that range.
The human keratin fibres treated via the process according to the invention are preferably the hair.
The expression "at least one" is equivalent to the expression "one or more".

### Composition A

The composition A according to the invention contains at least one peroxygenated salt, which can be advantageously chosen from ammonium persulfates, perborates and percarbonates perborates and percarbonates or alkali metal persulfates, perborates and percarbonates and also magnesium peroxide and mixtures of these compounds.

It advantageously comprises at least one persulfate as peroxygenated salt. Preferably, the persulfate(s) is (are) chosen from sodium persulfates, potassium persulfates and ammonium persulfates, and mixtures thereof.

The concentration of peroxygenated salts in the composition A in accordance with the invention generally ranges from 10% to 95% by weight, preferably from 30% to 90% by weight, better still from 50% to 85% by weight and even better still from 70% to 85% of the total weight of the composition A.

The composition A may be in the form of a paste or a powder.

The composition A according to the invention may also contain fillers such as clays, for instance kaolin, silica, binders such as vinylpyrrolidone, lubricants, for instance polyol stearates or alkali metal or alkaline-earth metal stearates, and also agents for controlling the release of oxygen, such as magnesium carbonate or magnesium oxide, colouring agents or matting agents such as titanium oxides.

### Composition B

### Basifying agents

As indicated previously, the composition B according to the invention comprises one or more basifying agents.
The basifying agent(s) may be inorganic or organic or hybrid.
For the purposes of the present invention, the term "inorganic compound" means any compound bearing in its structure one or more elements from columns 1 to 13 of the Periodic Table of Elements other than hydrogen.
According to one particular embodiment of the invention, the inorganic basifying agent contains one or more elements from columns 1 and 2 of the Periodic Table of Elements other than hydrogen.
In one preferred variant, the inorganic basifying agent has the following structure:

(Z₁^{x-})ₘ(Z₂^{y+})ₙ

in which:
Z₂ denotes a metal from columns 1 to 13 and preferably from column 1 or 2 of the Periodic Table of Elements, for instance sodium or potassium;
Z₁^{x-} denotes an anion chosen from the ions CO₃²⁻ , OH⁻, HCO₃²⁻ , SiO₃²⁻ , HPO₄²⁻ , PO₄³⁻ and B₄O₇²⁻, and preferably from the ions CO₃²⁻, OH⁻ and SiO₃²⁻;
x denotes 1, 2 or 3;
y denotes 1, 2, 3 or 4;
m and n denote, independently of each other, 1, 2, 3 or 4;
with nxy = m×x.
Preferably, the inorganic basifying agent corresponds to the following formula (Z₁^{x-}) ₘ(Z₂^{y+})ₙ, in which Z₂ denotes a metal from columns 1 and 2 of the Periodic Table of Elements; Z₁^{x-} denotes an anion chosen from the ions CO₃²⁻, OH⁻ and SiO₃²⁻, x is 1, y denotes 1 or 2, and m and n denote, independently of each other, 1 or 2 with nxy = m×x.
The inorganic basifying agent(s) can be chosen from aqueous ammonia, alkaline carbonates or bicarbonates such as sodium carbonate, potassium carbonate, sodium bicarbonate or potassium bicarbonate, sodium hydroxide (caustic soda), potassium hydroxide (potash), alkaline metasilicates, for instance sodium metasilicate or potassium metasilicate, and mixtures thereof, and more particularly alkaline carbonates.
The organic basifying agent(s) is (are) preferably chosen from organic amines with a pK_{b} at 25°C of less than 12, preferably less than 10 and even more advantageously less than 6. It should be noted that it is the pK_{b} corresponding to the function of highest basicity. In addition, the organic amines do not comprise an alkyl or alkenyl fatty chain comprising more than ten carbon atoms.
The organic basifying agent(s) is (are) chosen, for example, from alkanolamines, oxyethylenated and/or oxypropylenated ethylenediamines, amino acids and the compounds having formula (II) below: 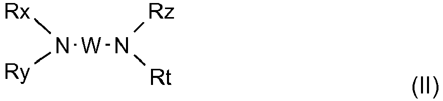 in which formula (II), W is a divalent C₁-C₆ alkylene radical optionally substituted with one or more hydroxyl groups or a C₁-C₆ alkyl radical, and/or optionally interrupted by one or more heteroatoms such as O, or NRu; Rx, Ry, Rz, Rt, Ru, which may be identical or different, represent a hydrogen atom, a C₁-C₆ alkyl or C₁-C₆ hydroxyalkyl radical, or a C₁-C₆ aminoalkyl radical.
Examples of amines having formula (II) that may be mentioned include 1,3-diaminopropane, 1,3-diamino-2-propanol, spermine and spermidine.
The term "alkanolamine" means an organic amine comprising a primary, secondary or tertiary amine function, and one or more linear or branched C₁-C₈ alkyl groups bearing one or more hydroxyl radicals.
The organic amines chosen from alkanolamines such as monoalkanolamines, dialkanolamines or trialkanolamines comprising one to three identical or different C₁-C₄ hydroxyalkyl radicals are in particular suitable for performing the invention. Among the compounds of this type, mention may be made of monoethanolamine (MEA), diethanolamine, triethanolamine, monoisopropanolamine, diisopropanolamine, N,N-dimethylethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethyl)aminomethane.
More particularly, the amino acids which can be used are of natural or synthetic origin, in their L, D or racemic form, and comprise at least one acid function chosen more particularly from carboxylic acid, sulfonic acid, phosphonic acid or phosphoric acid functions. The amino acids can be in the neutral or ionic form.
As amino acids that can be used in the present invention, mention may in particular be made of aspartic acid, glutamic acid, alanine, arginine, ornithine, citrulline, asparagine, carnitine, cysteine, glutamine, glycine, histidine, lysine, isoleucine, leucine, methionine, N-phenylalanine, proline, serine, taurine, threonine, tryptophan, tyrosine and valine.
Advantageously, the amino acids are basic amino acids comprising an additional amine function optionally included in a ring or in a ureido function.
Such basic amino acids are preferably chosen from those corresponding to formula (III) below: 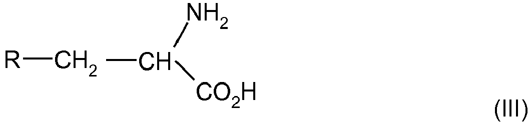 In which formula (III), R represents a group chosen from: 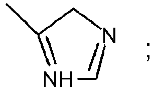 -(CH₂)₃NH₂;

-(CH₂)₂NH₂;

-(CH₂)₂NHCONH₂.

The compounds corresponding to formula (III) are histidine, lysine, arginine, ornithine and citrulline.
The organic amine can also be chosen from organic amines of heterocyclic type. Mention may in particular be made, in addition to histidine, already mentioned in the amino acids, of pyridine, piperidine, imidazole, triazole, tetrazole or benzimidazole. The organic amine can also be chosen from amino acid dipeptides. As amino acid dipeptides that can be used in the present invention, mention may in particular be made of carnosine, anserine and balenine.
The organic amine can also be chosen from compounds comprising a guanidine function. Mention may in particular be made, as amines of this type which can be used in the present invention, in addition to arginine, already mentioned as an amino acid, of creatine, creatinine, 1,1-dimethylguanidine, 1,1-diethylguanidine, glycocyamine, metformin, agmatine, N-amidinoalanine, 3-guanidinopropionic acid, 4-guanidinobutyric acid and 2-([amino(imino)methyl]amino)ethane-1-sulfonic acid. Hybrid compounds that may be mentioned include the salts of the amines mentioned previously with acids such as carbonic acid or hydrochloric acid.
Guanidine carbonate or monoethanolamine hydrochloride may in particular be used.

Preferably, the basifying agent(s) present in the composition B of the invention is (are) chosen from organic alkaline agents, such as alkanolamines, amino acids in neutral or ionic form, in particular basic amino acids, and preferably corresponding to those of formula (III), compounds comprising a guanidine function, and mixtures thereof.
Even more preferentially, the basifying agent(s) is (are) chosen from organic alkaline agents, preferably alkanolamines, in particular monoethanolamine (MEA).

Advantageously, the composition B according to the invention has a content of basifying agents agents ranging from 0.1% to 30% by weight and preferably from 1% to 20% by weight relative to the weight of said composition.

According to a first particular embodiment, the composition according to the invention does not use aqueous ammonia, or a salt thereof, as basifying agent.

According to a second embodiment, if the composition according to the invention was to use aqueous ammonia, or a salt thereof, as basifying agent, its content advantageously would not exceed 0.03% by weight (expressed as NH₃) and preferably would not exceed 0.01% by weight, relative to the weight of the composition of the invention.

Preferably, if the composition comprises aqueous ammonia, or a salt thereof, then the amount of basifying agent(s) other than the aqueous ammonia is greater than that of the aqueous ammonia (expressed as NH₃).

The composition B according to the present invention is aqueous.
It generally comprises from 5% to 60% by weight, better still from 20% to 58% by weight and even better still from 30% to 55% by weight of water relative to the total weight of the composition B.
It may optionally comprise one or more water-soluble organic solvents. Examples of water-soluble organic solvents that may be mentioned include C₁-C₄ lower alkanols, such as ethanol and isopropanol; aromatic alcohols such as benzyl alcohol or phenoxyethanol; polyols or polyol ethers such as ethylene glycol monomethyl, monoethyl and monobutyl ether, propylene glycol or ethers thereof such as propylene glycol monomethyl ether, butylene glycol, dipropylene glycol, and also diethylene glycol alkyl ethers, for instance diethylene glycol monoethyl ether or monobutyl ether, or alternatively glycerol; and also mixtures thereof.
The water-soluble organic solvents are preferably present in proportions of between 0.1% and 35% by weight approximately and even more preferentially between 1% and 20% by weight approximately, relative to the total weight of the composition B.

### Composition C

The composition C, also called "oxidizing composition", comprises at least one oxidizing agent.
The oxidizing agent(s) is (are) more particularly chemical oxidizing agents (as opposed to compositions in which the only oxidizing agent is atmospheric oxygen) and may be chosen from hydrogen peroxide, peroxygenated salts, for instance alkali metal or alkaline-earth metal persulfates, percarbonates and perborates, urea peroxide, polythionates, alkali metal bromates or ferricyanides, and peracids, and precursors thereof, or mixtures thereof. Hydrogen peroxide is preferred.
The oxidizing agent(s) generally represent(s) from 0.1% to 50% and preferably from 1% to 20% by weight relative to the total weight of the composition C.

According to one particular embodiment of the invention, when the oxidizing agent is hydrogen peroxide, the oxidizing composition C comprises one or more stabilizers of aqueous hydrogen peroxide.
Examples of stabilizers of aqueous hydrogen peroxide that may be mentioned in particular include alkali metal or alkaline-earth metal pyrophosphates, such as tetrasodium pyrophosphate, alkali metal or alkaline-earth metal stannates, phenacetin or oxyquinoline acid salts, for instance oxyquinoline sulfate. Preferably, one or more stannates are used optionally in combination with one or more pyrophosphates.

The stabilizer(s) of aqueous hydrogen peroxide generally represent(s) from 0.0001% to 5% by weight and preferably from 0.01% to 2% by weight relative to the total weight of the composition C.
The oxidizing composition C according to the present invention is preferably an aqueous composition comprising water and/or organic solvents in the contents indicated above.

The pH of the oxidizing composition C is less than or equal to 5 and more particularly ranges from 1.5 to 4.5 and preferably from 2 to 3.5.
The pH of the composition C may be adjusted using acidifying agents, for instance phosphoric acid, hydrochloric acid, acetic acid, lactic acid, boric acid or citric acid. Preferably, the pH of the composition according to the invention is adjusted using phosphoric acid.

The composition C comprises at least 3% by weight of fatty substances as described hereinafter, preferably at least 5% by weight, better still at least 10% by weight, even better still at least 15% by weight, more preferably at least 20% by weight, even better still at least 25% by weight of fatty substances relative to its total weight. Preferably, the composition C comprises an oil as fatty substance, in the contents indicated above for the composition B.

The oxidizing composition C according to the present invention is preferably an aqueous composition comprising water and/or organic solvents in the contents indicated above.
The pH of the oxidizing composition C is less than or equal to 5 and more particularly ranges from 1.5 to 4.5 and preferably from 2 to 3.5.
The pH of the composition C may be adjusted using acidifying agents, for instance phosphoric acid, hydrochloric acid, acetic acid, lactic acid, boric acid or citric acid. Preferably, the pH of the composition according to the invention is adjusted using phosphoric acid.

### Fatty substances

The bleaching composition resulting of the mixing of compositions A, B and C according to the invention comprises one or more fatty substances in a content greater than or equal to 10% by weight.
The term "fatty substance" means an organic compound that is insoluble in water at ordinary ambient temperature (25°C) and at atmospheric pressure (760 mmHg) (solubility of less than 5%, preferably less than 1% and even more preferentially less than 0.1%). They exhibit, in their structure, at least one hydrocarbon-based chain containing at least 6 carbon atoms or a sequence of at least two siloxane groups. In addition, the fatty substances are generally soluble in organic solvents under the same temperature and pressure conditions, for instance chloroform, ethanol, benzene, liquid petroleum jelly or decamethylcyclopentasiloxane. These fatty substances are neither polyoxyethylenated nor polyglycerolated.
More particularly, the fatty substances are chosen from C₆-C₁₆ hydrocarbons, hydrocarbons containing more than 16 carbon atoms, non-silicone oils of animal origin, plant oils of triglyceride type, synthetic triglycerides, fluoro oils, fatty alcohols, fatty acid and/or fatty alcohol esters other than triglycerides and plant waxes, non-silicone waxes and silicones.
It is recalled that, for the purposes of the invention, the fatty alcohols, fatty esters and fatty acids more particularly contain one or more linear or branched, saturated or unsaturated hydrocarbon-based groups containing 6 to 30 carbon atoms, optionally substituted, in particular with one or more hydroxyl groups (in particular 1 to 4). If they are unsaturated, these compounds may comprise one to three conjugated or unconjugated carbon-carbon double bonds.

As regards the C₆-C₁₆ hydrocarbons, they are linear, branched or optionally cyclic, and are preferably alkanes. Examples that may be mentioned include hexane, dodecane and isoparaffins such as isohexadecane and isodecane.
Mention may be made, as hydrocarbon-based oils of animal origin, of perhydrosqualene.
The triglyceride oils of plant or synthetic origin are preferably chosen from liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol® 810, 812 and 818 by the company Dynamit Nobel, jojoba oil and shea butter oil.
The linear or branched hydrocarbons of mineral or synthetic origin containing more than 16 carbon atoms are preferably chosen from liquid paraffins, petroleum jelly, liquid petroleum jelly, polydecenes and hydrogenated polyisobutene such as Parleam®.
The fluoro oils may be chosen from perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-trifluoromethyl perfluoromorpholine sold under the name PF 5052® by the company 3M.
The fatty alcohols that may be used in the cosmetic composition are saturated or unsaturated, and linear or branched, and contain from 6 to 30 carbon atoms and more particularly from 8 to 30 carbon atoms. Mention may be made, for example, of cetyl alcohol, stearyl alcohol and their mixture (cetearyl alcohol), octyldodecanol, 2-butyloctanol, 2-hexyldecanol, 2-undecylpentadecanol, oleyl alcohol or linoleyl alcohol.
The wax(es) that may be used in the cosmetic composition are chosen especially from carnauba wax, candelilla wax, esparto wax, paraffin wax, ozokerite, plant waxes, for instance olive wax, rice wax, hydrogenated jojoba wax or the absolute waxes of flowers such as the essential wax of blackcurrant blossom sold by Bertin (France), animal waxes, for instance beeswaxes, or modified beeswaxes (cera bellina); other waxes or waxy starting materials that may be used according to the invention are especially marine waxes such as the product sold by Sophim as M82, and polyethylene waxes or polyolefin waxes in general.
The fatty acids that may be used in the cosmetic composition may be saturated or unsaturated and contain from 6 to 30 carbon atoms and in particular from 9 to 30 carbon atoms. They are more particularly chosen from myristic acid, palmitic acid, stearic acid, behenic acid, oleic acid, linoleic acid, linolenic acid and isostearic acid. As regards the esters of fatty acids and/or of fatty alcohols, which are advantageously different from the triglycerides mentioned above, mention may be made in particular of esters of saturated or unsaturated, linear or branched C₁-C₂₆ aliphatic mono- or polyacids and of saturated or unsaturated, linear or branched C₁-C₂₆ aliphatic mono- or polyalcohols, the total carbon number of the esters more particularly being greater than or equal to 10.
Among the monoesters, mention may be made of dihydroabietyl behenate; octyldodecyl behenate; isocetyl behenate; cetyl lactate; C₁₂-C₁₅ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononanoate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate. Still within the context of this variant, esters of C₄-C₂₂ dicarboxylic or tricarboxylic acids and of C₁-C₂₂ alcohols and esters of mono-, di- or tricarboxylic acids and of C₂-C₂₆ di-, tri-, tetra- or pentahydroxy alcohols may also be used.
Mention may in particular be made of: diethyl sebacate; diisopropyl sebacate; diisopropyl adipate; di(n-propyl) adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.
Among the esters mentioned above, it is preferred to use ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanoate or cetyl octanoate.
The composition may also comprise, as fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which contain at least 4 carbon atoms. These sugars can be monosaccharides, oligosaccharides or polysaccharides.
Mention may be made, as suitable sugars, for example, of sucrose (or saccharose), glucose, galactose, ribose, fucose, maltose, fructose, mannose, arabinose, xylose, lactose and their derivatives, in particular alkyl derivatives, such as methyl derivatives, for example methylglucose.
The esters of sugars and of fatty acids may be chosen in particular from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.
The esters according to this variant can also be chosen from mono-, di-, tri- and tetraesters, polyesters, and mixtures thereof.
These esters can, for example, be oleates, laurates, palmitates, myristates, behenates, cocoates, stearates, linoleates, linolenates, caprates, arachidonates or mixtures thereof, such as, in particular, oleate/palmitate, oleate/stearate or palmitate/stearate mixed esters.
More particularly, use is made of mono- and diesters and in particular mono- or di-oleate, -stearate, -behenate, -oleate/palmitate, -linoleate, -linolenate or-oleate/stearate of sucrose, of glucose or of methylglucose.
Mention may be made, by way of example, of the product sold under the name Glucate® DO by Amerchol, which is a methylglucose dioleate.

Examples of esters or mixtures of esters of sugar and of fatty acid that may also be mentioned include:
- the products sold under the names F160, F140, F110, F90, F70 and SL40 by the company Crodesta, respectively denoting sucrose palmitostearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;
- the products sold under the name Ryoto Sugar Esters, for example referenced B370 and corresponding to sucrose behenate formed of 20% monoester and 80% diester, triester and polyester;
- the sucrose monopalmitate/stearate-dipalmitate/stearate sold by Goldschmidt under the name Tegosoft® PSE.
The silicones that can be used in the cosmetic composition according to the present invention are volatile or non-volatile, cyclic, linear or branched silicones, which are unmodified or modified by organic groups, having a viscosity from 5×10⁻⁶ to 2.5 m²/s at 25°C, and preferably 1×10⁻⁵ to 1 m²/s.
The silicones which can be used in accordance with the invention can be in the form of oils, waxes, resins or gums.
Preferably, the silicone is chosen from polydialkylsiloxanes, in particular polydimethylsiloxanes (PDMSs), and organomodified polysiloxanes comprising at least one functional group chosen from poly(oxyalkylene) groups, amino groups and alkoxy groups.
Organopolysiloxanes are defined in greater detail in Walter Noll's Chemistry and Technology of Silicones (1968), Academic Press. They can be volatile or non-volatile.
When they are volatile, the silicones are more particularly chosen from those having a boiling point of between 60°C and 260°C, and more particularly still from:
(i) cyclic polydialkylsiloxanes containing from 3 to 7 and preferably from 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, and Silbione® 70045 V5 by Rhodia, and mixtures thereof.

Mention may also be made of cyclocopolymers of the dimethylsiloxane/methylalkylsiloxane type, such as Volatile Silicone® FZ 3109 sold by the company Union Carbide, of formula:

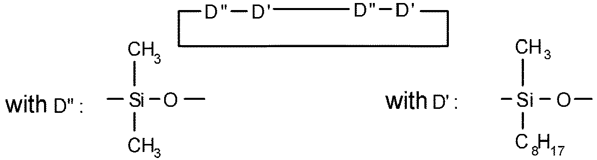

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane; (ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones coming within this category are also described in the paper published in Cosmetics and Toiletries, Vol. 91, Jan. 76, pp. 27-32, Todd & Byers, "Volatile Silicone Fluids for Cosmetics".
Use is preferably made of non-volatile polydialkylsiloxanes, polydialkylsiloxane gums and resins, polyorganosiloxanes modified with the organofunctional groups above, and mixtures thereof.
These silicones are more particularly chosen from polydialkylsiloxanes, among which mention may be made mainly of polydimethylsiloxanes having trimethylsilyl end groups. The viscosity of the silicones is measured at 25°C according to Standard ASTM 445 Appendix C.
Mention may be made, among these polydialkylsiloxanes, without implied limitation, of the following commercial products:
- the Silbione® oils of the 47 and 70 047 series or the Mirasil® oils sold by Rhodia, such as, for example, the oil 70 047 V 500 000;
- the oils of the Mirasil® series sold by Rhodia;
- the oils of the 200 series from the company Dow Corning, such as DC200 with a viscosity of 60 000 mm²/s;
- the Viscasil® oils from General Electric and certain oils of the SF series (SF 96, SF 18) from General Electric.
Mention may also be made of polydimethylsiloxanes having dimethylsilanol end groups known under the name dimethiconol (CTFA), such as the oils of the 48 series from Rhodia.
In this category of polydialkylsiloxanes, mention may also be made of the products sold under the names Abil Wax® 9800 and 9801 by the company Goldschmidt, which are polydi(C₁-C₂₀)alkylsiloxanes.
The silicone gums that may be used in accordance with the invention are in particular polydialkylsiloxanes and preferably polydimethylsiloxanes with high number-average molecular weights of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane, tridecane or mixtures thereof.
Products which can be used more particularly in accordance with the invention are mixtures such as:
- the mixtures formed from a polydimethylsiloxane hydroxylated at the chain end, or dimethiconol (CTFA), and from a cyclic polydimethylsiloxane, also known as cyclomethicone (CTFA), such as the product Q2 1401 sold by Dow Corning;
- the mixtures of a polydimethylsiloxane gum and of a cyclic silicone, such as the product SF 1214 Silicone Fluid from General Electric; this product is an SF 30 gum corresponding to a dimethicone, having a number-average molecular weight of 500 000, dissolved in the oil SF 1202 Silicone Fluid corresponding to decamethylcyclopentasiloxane;
- the mixtures of two PDMSs with different viscosities, and more particularly of a PDMS gum and a PDMS oil, such as the product SF 1236 from General Electric. The product SF 1236 is the mixture of an SE 30 gum defined above, with a viscosity of 20 m²/s, and of an SF 96 oil with a viscosity of 5×10⁻⁶ m²/s. This product preferably comprises 15% of SE 30 gum and 85% of an SF 96 oil.
The organopolysiloxane resins which can be used in accordance with the invention are crosslinked siloxane systems containing the following units:

R₂SiO_{2/2}, R₃SiO_{1/2}, RSiO_{3/2} and SiO_{4/2},

in which R represents an alkyl containing 1 to 16 carbon atoms. Among these products, those that are particularly preferred are those in which R denotes a C₁-C₄ lower alkyl group, more particularly methyl.
Mention may be made, among these resins, of the product sold under the name Dow Corning 593 or those sold under the names Silicone Fluid SS 4230 and SS 4267 by General Electric, which are silicones of dimethyl/trimethylsiloxane structure.
Mention may also be made of the resins of the trimethylsiloxysilicate type, sold in particular under the names X22-4914, X21-5034 and X21-5037 by Shin-Etsu.
The organomodified silicones that can be used in accordance with the invention are silicones as defined above and comprising in their structure one or more organofunctional groups attached via a hydrocarbon-based group.
Besides the silicones described above, the organomodified silicones may be polydiarylsiloxanes, especially polydiphenylsiloxanes, and polyalkylarylsiloxanes functionalized by the organofunctional groups mentioned previously.
The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.
Among these polyalkylarylsiloxanes, examples that may be mentioned include the products sold under the following names:
- Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.
Mention may be made, among the organomodified silicones, of polyorganosiloxanes comprising:
- polyethyleneoxy and/or polypropyleneoxy groups optionally comprising C₆-C₂₄ alkyl groups, such as the products known as dimethicone copolyol sold by the company Dow Corning under the name DC 1248 or the oils Silwet® L 722, L 7500, L 77 and L 711 by the company Union Carbide, and the (C₁₂)alkylmethicone copolyol sold by the company Dow Corning under the name Q2 5200;
- substituted or unsubstituted amino groups, such as the products sold as GP 4 Silicone Fluid and GP 7100 by Genesee or the products sold as Q2 8220 and Dow Corning 929 or 939 by Dow Corning. The substituted amine groups are, in particular, C1-C4 aminoalkyl groups;
- alkoxylated groups, such as the product sold under the name Silicone Copolymer F-755 by SWS Silicones, and Abil Wax® 2428, 2434 and 2440 by Goldschmidt.

Preferably, the fatty substances do not comprise any C₂-C₃ oxyalkylene units or any glycerol units.
More particularly, the fatty substances are chosen from compounds that are liquid or pasty at ambient temperature and atmospheric pressure.
Preferably, the bleaching composition comprises at least one fatty substance that is liquid at a temperature of 25°C and at atmospheric pressure (or oil).

The fatty substance(s) is (are) advantageously chosen from C₆-C₁₆ hydrocarbons, hydrocarbons containing more than 16 carbon atoms, non-silicone oils of animal origin, plant oils of triglyceride type, synthetic triglycerides, fluoro oils, fatty alcohols, fatty acid and/or fatty alcohol esters other than triglycerides and plant waxes, non-silicone waxes and silicones, and mixtures thereof.
Preferably, the fatty substance(s) is (are) chosen from liquid petroleum jelly, polydecenes, fatty alcohols, liquid fatty acid and/or fatty alcohol esters, and their mixtures.
Even more preferentially, the fatty substances are chosen from liquid petroleum jelly and fatty alcohols, and mixtures thereof.

The bleaching composition according to the invention comprises one or more fatty substances in a content greater than or equal to 10% by weight, preferably greater or equal to 12% by weight, even better still greater than or equal to 15% by weight, even more preferably greater than or equal to 20% by weight, and better still greater than or equal to 25% by weight relative to the weight of the bleaching composition. The fatty substance content in the bleaching composition may range, for example, from 10% to 40% by weight, better still from 12% to 35% by weight, preferably from 15% to 30% by weight and even better still from 20% to 25% by weight relative to the total weight of the bleaching composition.

The fatty substance content in the bleaching composition may range, for example, from 10% to 50% by weight, better still from 12% to 40% by weight, preferably from 15% to 35% by weight and even better still from 20% to 30% by weight relative to the total weight of the bleaching composition.
According to one embodiment, the bleaching composition comprises at least 10%, preferably at least 12%, by weight, of oil as fatty substance.
The content of oil(s) in the bleaching composition may range, for example, from 10% to 40% by weight, better still from 12% to 30% by weight, preferably from 14% to 25% by weight, even better still from 14% to 20% by weight and more preferably from 14% to 18% by weight relative to the total weight of the bleaching composition.

The fatty substance content in each composition A, B and/or C of the invention may range from 5% to 90%, preferably from 10% to 75%, even more preferably from 20% to 60%, better still from 30% to 55% and even better still from 35% to 55% relative to the total weight of each composition.

According to one embodiment, the composition B and/or the composition C comprises at least 10% by weight, preferably at least 20% by weight, better still at least 25% by weight and even better still at least 30% by weight of fatty substance relative to its total weight.

Each composition A, B and/or C of the invention can also comprise additional compounds conventionally used in the cosmetics industry. These compounds can in particular be chosen from inorganic or organic thickeners, and in particular anionic, cationic, non-ionic or amphoteric, associative or non-associative thickening polymers, fillers such as clays, binders such as vinylpyrrolidone, lubricants, for instance polyol stearates or alkali metal or alkaline-earth metal stearates, hydrophilic or hydrophobic silicas, pigments, dyes, matting agents, such as titanium oxides, or else anionic, non-ionic, cationic, amphoteric or zwitterionic surfactants, antioxidants, penetrating agents, sequestering agents, buffers, dispersants, film-forming agents, preservatives, opacifiers, vitamins, fragrances, anionic, cationic, non-ionic, amphoteric or zwitterionic polymers, ceramides, and conditioning agents such as, for example, modified or unmodified, volatile or non-volatile silicones.

The surfactants may be chosen from anionic, amphoteric, zwitterionic, cationic or non-ionic surfactants, and preferentially non-ionic surfactants.
Examples of non-ionic surfactants that may be used in the composition used according to the invention are described, for example, in the Handbook of Surfactants by M.R. Porter, published by Blackie & Son (Glasgow and London), 1991, pp. 116-178.
The amount of the surfactant(s) in each composition A, B or C preferably ranges from 0.1% to 50% by weight and even better still from 0.5% to 20% by weight relative to the total weight of each composition.

In particular, the surfactants may be oxyalkylenated non-ionic surfactants, which preferably comprise at least 10 oxyalkylene groups, preferably at least 15 oxyalkylene groups.
They can in particular have a number of oxyalkylene groups ranging from 10 to 50, preferably from 15 to 30.
The oxyalkylene groups are preferably oxyethylene and/or oxypropylene groups. Advantageously, the oxyalkylene groups of the non-ionic surfactant(s) used in the invention are oxyethylene groups.

Examples of oxyalkylenated non-ionic surfactants that may be mentioned include:
- oxyalkylenated (C₈-C₂₄)alkylphenols,
- saturated or unsaturated, linear or branched, oxyalkylenated C₈-C₃₀, preferably C₁₂-C₂₂, fatty alcohols,
- saturated or unsaturated, linear or branched, oxyalkylenated C₈-C₃₀ amides,
- esters of saturated or unsaturated, linear or branched, C₈-C₃₀ acids and of polyethylene glycols,
- polyoxyethylenated esters of saturated or unsaturated, linear or branched, C₈-C₃₀ acids and of sorbitol,
- saturated or unsaturated oxyethylenated plant oils,
   and mixtures thereof.

Preferably, the oxyalkylenated non-ionic surfactant is chosen from saturated or unsaturated, linear or branched, oxyalkylenated, preferably oxyethylenated, C₈-C₃₀, preferably C₁₂-C₂₂, fatty alcohols, for instance the products of addition of ethylene oxide with lauryl alcohol, in particular those comprising from 10 to 50 oxyethylene groups and more particularly those comprising from 10 to 30 oxyethylene groups (CTFA names Laureth-10 to Laureth-30); the products of addition of ethylene oxide with behenyl alcohol, in particular those comprising from 10 to 50 oxyethylene groups (CTFA names Beheneth-9 to Beheneth-50); the products of addition of ethylene oxide with cetearyl alcohol (mixture of cetyl alcohol and stearyl alcohol), in particular those comprising from 10 to 30 oxyethylene groups (CTFA names Ceteareth-10 to Ceteareth-30); the products of addition of ethylene oxide with cetyl alcohol, in particular those comprising from 10 to 30 oxyethylene groups (CTFA names Ceteth-10 to Ceteth-30); the products of addition of ethylene oxide with stearyl alcohol, in particular those comprising from 10 to 30 oxyethylene groups (CTFA names Steareth-10 to Steareth-30); the products of addition of ethylene oxide with isostearyl alcohol, in particular those comprising from 10 to 50 oxyethylene groups (CTFA names Isosteareth-10 to Isosteareth-50); the products of addition of ethylene oxide with oleocetyl alcohol, in particular those comprising from 10 to 50 oxyethylene groups (CTFA names oleth-10 to oleth-50), and mixtures thereof.
According to one embodiment, the products of addition of ethylene oxide with stearyl alcohol, in particular those comprising from 10 to 30 oxyethylene groups (CTFA names Steareth-10 to Steareth-30), are in particular used.

The content of oxyalkylenated non-ionic surfactants in each composition A, B and/or C of the process according to the invention can range from 0.1% to 20% by weight, preferably from 0.5% to 10% by weight and better still from 1% to 5% by weight relative to the total weight of each composition.

Needless to say, those skilled in the art will take care to select this or these optional additional compound(s) such that the advantageous properties intrinsically associated with the composition in accordance with the invention are not, or are not substantially, adversely affected by the envisaged addition(s).

Each composition B and/or C of the process according to the invention may be in various forms, such as in the form of a cream, a gel, a milk, a lotion or a mousse, or in any other form that is suitable for treating keratin fibres, and in particular human keratin fibres such as the hair.

The bleaching process comprises a step of applying to the keratin fibres the bleaching composition resulting from the mixing of the compositions A, B and C described above. In general, the leave-on time of the composition on the fibres ranges from 1 to 60 minutes approximately and preferably from 1 to 30 minutes approximately.
Usually, the temperature at which the composition is applied is from about 15 to 80°C and preferably from 15 to 40°C.
Once the desired bleaching has been obtained, the bleaching mixture is usually removed by rinsing the fibres with water, preferably followed by washing them at least once with a shampoo, and then optionally drying them.

The example that follows illustrates the invention without, however, being limiting in nature.

### EXAMPLE: Bleaching process

The following compositions A, B and C were prepared (amounts in g%):

**Composition A (Anhydrous powder)**

| | |
|---|---|
| Sodium persulfate | 10 |
| Potassium persulfate | 70 |
| Ethylenediaminetetraacetic acid | 1 |
| Anhydrous sodium metasilicate (Silmaco) | 10 |
| Hydrated precipitated silica (4-25 microns) | 2 |
| (Standard Levilite from Ceca) | |
| Kaolinite | 2 |
| (Kaolin Supreme from Imerys) | |
| Titanium oxide (CI: 77891) | 5 |
| (Hombitan FF pharma from Sachtleben) | |

**Aqueous Composition B**

| | |
|---|---|
| Diethylenetriaminepentaacetic acid, pentasodium salt as an aqueous 40% solution | 2 |
| (Dissolvine D 40 from Akzo Nobel) | |
| Pure monoethanolamine | 6.7 |
| Fumed silica of hydrophobic nature | 1.2 |
| (Aerosil R 972 from Evonik) | |
| Glycol distearate | 2 |
| (Tegin BL 315 from Evonik) | |
| Cetearyl alcohol (C₁₆/C₁₈ 50/50) | 11.5 |
| (Lanette O Or from Cognis) | |
| Fragrance | 0.5 |
| Dimethyldiallylammonium chloride/acrylic acid copolymer (80/20) as a protected aqueous solution | 3.7 |
| (Merquat 280 from Nalco) | |
| Carboxyvinyl polymer synthesized in the ethyl acetate/cyclohexane mixture | 0.4 |
| (Carbopol 980 from Lubrizol) | |
| Deionized water | 37.8 |
| Propylene glycol | 10 |
| Lauric acid | 3 |
| Oxyethylenated lauryl alcohol (12 OE) (Ewopal 12 from Evonik) | 7 |
| Oxyethylenated decyl alcohol (3 OE) (Emulgin BL 309 from Cognis) | 10 |
| Oxyethylenated oleocetyl alcohol (30 OE) | 4 |
| (Emulgin O 30 from Cognis) | |

**Composition C (oxidizing agent)**

| | |
|---|---|
| Diethylenetriaminepentaacetic acid, pentasodium salt as an aqueous 40% solution | 0.15 |
| Hydrogen peroxide as a 50% solution (200 vol. aqueous hydrogen peroxide solution) | 18 |
| Disodium tin hexahydroxide | 0.04 |
| Phosphoric acid | qs |
| Tetrasodium pyrophosphate.10 H₂O | 0.03 |
| Liquid petroleum jelly | 25 |
| (Marcol N82 from Exxonmobil) | |
| Poly[(dimethyliminio)-1,3-propanediyl(dimethyliminio)-1,6-hexanediyl dichloride] as an aqueous 60% solution (Ionene G from Chimex) | 0.25 |
| Non-stabilized polydimethyldiallylammonium chloride at 40% in water | 0.5 |
| Deionized water | 43.13 |
| Glycerol | 0.5 |
| Cetearyl alcohol (30/70 C₁₆/C₁₈) (Lanette D from Cognis) | 6 |
| Oxyethylenated stearyl alcohol (20 OE) | 5 |
| (Tego alkanol S20 P from Evonik) | |
| Protected oxyethylenated rapeseed acid amide (4 OE) (Amidet N from Kao) | 1.3 |
| Vitamin E: DL-Alpha-Tocopherol | 0.1 |

At the time of use, the following are mixed (by weight):
- 22 g of the composition A
- 25 g of the composition B,
- 75 g of the composition C.

The three compositions mix easily together, and the mixture obtained does not give off an unpleasant odour and is applied uniformly to the hair. Good bleaching of the hair is obtained.

## Claims

1. Multi-compartment device comprising:
- at least one anhydrous composition A comprising at least one peroxygenated salt,
- at least one aqueous composition B comprising at least one basifying agent, and at least one oxyalkylenated non-ionic surfactant chosen from saturated or unsaturated, linear or branched, oxyalkylenated C₈-C₃₀ fatty alcohols chosen from the products of addition of ethylene oxide with lauryl alcohol comprising from 10 to 50 oxyethylene groups; the products of addition of ethylene oxide with behenyl alcohol comprising from 10 to 50 oxyethylene groups; the products of addition of ethylene oxide with cetearyl alcohol (mixture of cetyl alcohol and stearyl alcohol) comprising from 10 to 30 oxyethylene groups; the products of addition of ethylene oxide with cetyl alcohol comprising from 10 to 30 oxyethylene groups; the products of addition of ethylene oxide with stearyl alcohol comprising from 10 to 30 oxyethylene groups; the products of addition of ethylene oxide with isostearyl alcohol comprising from 10 to 50 oxyethylene groups; the products of addition of ethylene oxide with oleocetyl alcohol comprising from 10 to 50 oxyethylene groups, and mixtures thereof; and
- at least one composition C comprising at least one oxidizing agent and at least 3% by weight of fatty substance relative to the total weight of said composition,
- said compositions being packaged separately and having a fatty substance content such that the total fatty substance content in the mixture of the compositions A, B and C is greater than or equal to 10% by weight of said mixture.

2. Device according to the preceding claim, **characterized in that** the peroxygenated salt is chosen from ammonium persulfates, perborates and percarbonates or alkali metal persulfates, perborates and percarbonates and also magnesium peroxide and mixtures of these compounds.

3. Device according to Claim 1 or 2, **characterized in that** the concentration of peroxygenated salts in the composition A ranges from 10% to 95% by weight, preferably from 30% to 90% by weight, better still from 50% to 85% by weight and even better still from 70% to 85% of the total weight of the composition A.

4. Device according to one of the preceding claims, **characterized in that** the basifying agent(s) in the composition B is (are) chosen from inorganic basifying agents such as aqueous ammonia, alkaline carbonates or bicarbonates, alkaline metasilicates, and mixtures thereof.

5. Device according to one of the preceding claims, **characterized in that** the basifying agent(s) in the composition B is (are) chosen from organic alkaline agents and preferably from alkanolamines, in particular monoethanolamine (MEA).

6. Device according to one of the preceding claims, **characterized in that** the composition B has a content of basifying agents ranging from 0.1% to 30% by weight and preferably from 1% to 20% by weight relative to the weight of said composition.

7. Device according to one of the preceding claims, **characterized in that** the oxidizing agent present in the composition C is hydrogen peroxide.

8. Device according to one of the preceding claims, **characterized in that** the oxidizing agent represents from 0.1% to 50% and preferably from 1% to 20% by weight relative to the total weight of the composition C.

9. Device according to one of the preceding claims, **characterized in that** the fatty substances are chosen from C₆-C₁₆ hydrocarbons, hydrocarbons containing more than 16 carbon atoms, non-silicone oils of animal origin, plant oils of triglyceride type, synthetic triglycerides, fluoro oils, fatty alcohols, fatty acid and/or fatty alcohol esters other than triglycerides and plant waxes, non-silicone waxes and silicones, and mixtures thereof.

10. Device according to one of the preceding claims, **characterized in that** the fatty substances are chosen from liquid petroleum jelly, polydecenes, fatty alcohols, and liquid esters of fatty acids and/or of fatty alcohols, and mixtures thereof.

11. Device according to one of the preceding claims, **characterized in that** the fatty substance content in the mixture of the compositions A, B and C is greater than or equal to 12% by weight, even better still greater than or equal to 15% by weight, even more preferably greater than or equal to 20% and better still greater than or equal to 25% by weight relative to the weight of the mixture of the compositions A, B and C.

12. Device according to any one of the preceding claims, **characterized in that** the fatty substance content ranges from 10% to 50% by weight, better still from 12% to 40% by weight, preferably from 15% to 35% by weight and even better still from 20% to 30% by weight relative to the total weight of the mixture of the compositions A, B and C.

13. Device according to any one of the preceding claims, **characterized in that** the mixture of the compositions A, B and C comprises at least 10% and preferably at least 12% by weight of oil.

14. Device according to one of the preceding claims, **characterized in that** the content of oil(s) in the mixture of the compositions A, B and C ranges from 10% to 40% by weight, better still from 12% to 30% by weight, preferably from 14% to 25% by weight and even better still from 14% to 20% by weight relative to the total weight of the mixture of the compositions A, B and C.

## Patentansprüche

1. Vorrichtung mit mehreren Kompartimenten, umfassend:
- mindestens eine wasserfreie Zusammensetzung A, umfassend mindestens ein peroxygeniertes Salz,
- mindestens eine wässrige Zusammensetzung B, umfassend mindestens ein Basifizierungsmittel und mindestens ein oxyalkyleniertes nichtionisches Tensid, das aus gesättigten oder ungesättigten, linearen oder verzweigten, oxyalkylenierten C₈-C₃₀-Fettalkoholen ausgewählt ist, die aus den Additionsprodukten von Ethylenoxid mit Laurylalkohol mit 10 bis 50 Oxyethylen-Gruppen, den Additionsprodukten von Ethylenoxid mit Behenylalkohol mit 10 bis 50 Oxyethylengruppen, den Additionsprodukten von Ethylenoxid mit Cetearylalkohol (Gemisch von Cetylalkohol und Stearylalkohol) mit 10 bis 30 Oxyethylen-Gruppen, den Additionsprodukten von Ethylenoxid mit Cetylalkohol mit 10 bis 30 Oxyethylen-Gruppen, den Additionsprodukten von Ethylenoxid mit Stearylalkohol mit 10 bis 30 Oxyethylen-Gruppen, den Additionsprodukten von Ethylenoxid mit Isostearylalkohol mit 10 bis 50 Oxyethylen-Gruppen, den Additionsprodukten von Ethylenoxid mit Oleocetylalkohol mit 10 bis 50 Oxyethylen-Gruppen und Mischungen davon ausgewählt sind; und
- mindestens eine Zusammensetzung C, umfassend mindestens ein Oxidationsmittel und mindestens 3 Gew.-% Fettsubstanz, bezogen auf das Gesamtgewicht der Zusammensetzung,
- wobei die Zusammensetzungen separat verpackt sind und einen solchen Fettsubstanzgehalt haben, dass der gesamte Fettsubstanzgehalt in der Mischung der Zusammensetzungen A, B und C größer oder gleich 10 Gew.-% der Mischung ist.

2. Vorrichtung nach dem vorhergehenden Anspruch, **dadurch gekennzeichnet, dass** das peroxygenierte Salz aus Ammoniumpersulfaten, -perboraten und -percarbonaten oder Alkalimetallpersulfaten, -perboraten und -percarbonaten sowie Magnesiumperoxid und Mischungen dieser Verbindungen ausgewählt ist.

3. Vorrichtung nach Anspruch 1 oder 2, **dadurch gekennzeichnet, dass** die Konzentration an peroxygenierten Salzen in der Zusammensetzung A im Bereich von 10 bis 95 Gew.-%, vorzugsweise von 30 bis 90 Gew.-%, noch besser von 50 bis 85 Gew.-% und sogar noch besser von 70 bis 85 Gew.-% des Gesamtgewichts der Zusammensetzung A liegt.

4. Vorrichtung nach einem der vorhergehenden Ansprüche, **dadurch gekennzeichnet, dass** das Basifizierungsmittel bzw. die Basifizierungsmittel in der Zusammensetzung B aus anorganischen Basifizierungsmitteln wie wässrigem Ammoniak, Alkalicarbonaten oder -hydrogencarbonaten, Alkalimetasilikaten und Mischungen davon ausgewählt ist bzw. sind.

5. Vorrichtung nach einem der vorhergehenden Ansprüche, **dadurch gekennzeichnet, dass** das Basifizierungsmittel bzw. die Basifizierungsmittel in der Zusammensetzung B aus organischen alkalischen Mitteln und vorzugsweise aus Alkanolaminen, insbesondere Monoethanolamin (MEA), ausgewählt ist bzw. sind.

6. Vorrichtung nach einem der vorhergehenden Ansprüche, **dadurch gekennzeichnet, dass** die Zusammensetzung B einen Gehalt an Basifizierungsmitteln im Bereich von 0,1 bis 30 Gew.-% und vorzugsweise von 1 bis 20 Gew.-%, bezogen auf das Gewicht der Zusammensetzung, aufweist.

7. Vorrichtung nach einem der vorhergehenden Ansprüche, **dadurch gekennzeichnet, dass** es sich bei dem Oxidationsmittel in Zusammensetzung C um Wasserstoffperoxid handelt.

8. Vorrichtung nach einem der vorhergehenden Ansprüche, **dadurch gekennzeichnet, dass** das Oxidationsmittel 0,1 bis 50 Gew.-% und vorzugsweise 1 bis 20 Gew.-%, bezogen auf das Gesamtgewicht der Zusammensetzung C, ausmacht.

9. Vorrichtung nach einem der vorhergehenden Ansprüche, **dadurch gekennzeichnet, dass** die Fettsubstanzen aus C₆-C₁₆-Kohlenwasserstoffen, Kohlenwasserstoffen mit mehr als 16 Kohlenstoffatomen, Nichtsilikonölen tierischer Herkunft, Pflanzenölen vom Triglycerid-Typ, synthetischen Triglyceriden, Fluorölen, Fettalkoholen, Fettsäure- und/oder Fettalkoholestern, die von Triglyceriden verschieden sind, und Pflanzenwachsen, Nichtsilikonwachsen und Silikonen und Mischungen davon ausgewählt sind.

10. Vorrichtung nach einem der vorhergehenden Ansprüche, **dadurch gekennzeichnet, dass** die Fettsubstanzen aus Vaselineöl, Polydecenen, Fettalkoholen und flüssigen Estern von Fettsäuren und/oder Fettalkoholen und Mischungen davon ausgewählt sind.

11. Vorrichtung nach einem der vorhergehenden Ansprüche, **dadurch gekennzeichnet, dass** der Fettsubstanzgehalt in der Mischung der Zusammensetzungen A, B und C größer oder gleich 12 Gew.-%, sogar noch besser größer oder gleich 15 Gew.-%, noch weiter bevorzugt größer oder gleich 20 Gew.-% und noch besser größer oder gleich 25 Gew.-%, bezogen auf die Mischung der Zusammensetzungen A, B und C, ist.

12. Vorrichtung nach einem der vorhergehenden Ansprüche, **dadurch gekennzeichnet, dass** der Fettsubstanzgehalt im Bereich von 10 bis 50 Gew.-%, noch besser von 12 bis 40 Gew.-%, vorzugsweise von 15 bis 35 Gew.-% und noch besser von 20 bis 30 Gew.-%, bezogen auf das Gesamtgewicht der Mischung der Zusammensetzungen A, B und C, liegt.

13. Vorrichtung nach einem der vorhergehenden Ansprüche, **dadurch gekennzeichnet, dass** die Mischung der Zusammensetzungen A, B und C mindestens 10 Gew.-% und vorzugsweise mindestens 12 Gew.-% Öl umfasst.

14. Vorrichtung nach einem der vorhergehenden Ansprüche, **dadurch gekennzeichnet, dass** der Gehalt an Öl(en) in der Mischung der Zusammensetzungen A, B und C im Bereich von 10 bis 40 Gew.-%, noch besser von 12 bis 30 Gew.-%, vorzugsweise von 14 bis 25 Gew.-% und sogar noch besser von 14 bis 20 Gew.-%, bezogen auf das Gesamtgewicht der Mischung der Zusammensetzungen A, B und C, liegt.

## Revendications

1. Dispositif à compartiments multiples comprenant :
- au moins une composition anhydre A comprenant au moins un sel peroxygéné,
- au moins une composition aqueuse B comprenant au moins un agent basifiant, et au moins un tensioactif non ionique oxyalkyléné choisi parmi des alcools gras en C₈-C₃₀ oxyalkylénés saturés ou insaturés, linéaires ou ramifiés choisis parmi les produits d'addition d'oxyde d'éthylène avec l'alcool laurylique comprenant de 10 à 50 groupes oxyéthylène ; les produits d'addition d'oxyde d'éthylène avec l'alcool béhénylique comprenant de 10 à 50 groupes oxyéthylène ; les produits d'addition d'oxyde d'éthylène avec l'alcool cétéarylique (mélange d'alcool cétylique et d'alcool stéarylique) comprenant de 10 à 30 groupes oxyéthylène ; les produits d'addition d'oxyde d'éthylène avec l'alcool cétylique comprenant de 10 à 30 groupes oxyéthylène ; les produits d'addition d'oxyde d'éthylène avec l'alcool stéarylique comprenant de 10 à 30 groupes oxyéthylène ; les produits d'addition d'oxyde d'éthylène avec l'alcool isostéarylique comprenant de 10 à 50 groupes oxyéthylène ; les produits d'addition d'oxyde d'éthylène avec l'alcool oléocétylique comprenant de 10 à 50 groupes oxyéthylène, et des mélanges de ceux-ci ; et
- au moins une composition C comprenant au moins un agent oxydant et au moins 3 % en poids de substance grasse par rapport au poids total de ladite composition, lesdites compositions étant conditionnées séparément et ayant une teneur en substance grasse telle que la teneur totale en substance grasse dans le mélange des compositions A, B et C est supérieur ou égal à 10 % en poids dudit mélange.

2. Dispositif selon la revendication précédente, **caractérisé en ce que** le sel peroxygéné est choisi parmi des persulfates, perborates et percarbonates d'ammonium ou des persulfates, perborates et percarbonates de métal alcalin et également le peroxyde de magnésium et des mélanges de ces composés.

3. Dispositif selon la revendication 1 ou 2, **caractérisé en ce que** la concentration de sels peroxygénés dans la composition A est dans la plage de 10 % à 95 % en poids, de préférence de 30 % à 90 % en poids, plus préférablement de 50 % à 85 % en poids et encore plus préférablement de 70 % à 85 % du poids total de la composition A.

4. Dispositif selon l'une des revendications précédentes, **caractérisé en ce que** le (s) agent(s) basifiant(s) dans la composition B est (sont) choisi(s) parmi des agents basifiants inorganiques tels que l'ammoniac aqueux, des carbonates ou bicarbonates alcalins, des métasilicates alcalins, et des mélanges de ceux-ci.

5. Dispositif selon l'une des revendications précédentes, **caractérisé en ce que** le(s) agent(s) basifiant(s) dans la composition B est (sont) choisi(s) parmi des agents alcalins organiques et, de préférence, parmi des alcanolamines, en particulier la monoéthanolamine (MEA).

6. Dispositif selon l'une des revendications précédentes, **caractérisé en ce que** la composition B a une teneur en agents basifiants dans la plage de 0,1 % à 30 % en poids et, de préférence, de 1 % à 20 % en poids par rapport au poids de ladite composition.

7. Dispositif selon l'une des revendications précédentes, **caractérisé en ce que** l'agent oxydant présent dans la composition C est le peroxyde d'hydrogène.

8. Dispositif selon l'une des revendications précédentes, **caractérisé en ce que** l'agent oxydant représente de 0,1 % à 50 % et de préférence de 1 % à 20 % en poids par rapport au poids total de la composition C.

9. Dispositif selon l'une des revendications précédentes, **caractérisé en ce que** les substances grasses sont choisies parmi des hydrocarbures en C₆-C₁₆, des hydrocarbures contenant pas plus de 16 atomes de carbone, des huiles non silicone d'origine animale, des huiles végétales de type triglycérides, des triglycérides synthétiques, des huiles fluoro, des alcools gras, des esters d'acide gras et/ou d'alcool gras autres que des triglycérides et des cires végétales, des cires non silicone et des silicones, et des mélanges de ceux-ci.

10. Dispositif selon l'une des revendications précédentes, **caractérisé en ce que** les substances grasses sont choisies parmi la gelée de pétrole liquide, des polydécènes, des alcools gras, et des esters d'acides gras et/ou alcools gras liquides, et des mélanges de ceux-ci.

11. Dispositif selon l'une des revendications précédentes, **caractérisé en ce que** la teneur en substance grasse dans le mélange des compositions A, B et C est supérieur ou égal à 12 % en poids, encore plus préférablement supérieur ou égal à 15 % en poids, encore plus préférablement supérieur ou égal à 20 % et plus préférablement supérieur ou égal à 25 % en poids par rapport au poids du mélange des compositions A, B et C.

12. Dispositif selon l'une quelconque des revendications précédentes, **caractérisé en ce que** la teneur en substance grasse est dans la plage de 10 % à 50 % en poids, plus préférablement de 12 % à 40 % en poids, de préférence de 15 % à 35 % en poids et encore plus préférablement de 20 % à 30 % en poids par rapport au poids total du mélange des compositions A, B et C.

13. Dispositif selon l'une quelconque des revendications précédentes, **caractérisé en ce que** le mélange des compositions A, B et C comprend au moins 10 % et de préférence au moins 12 % en poids d'huile.

14. Dispositif selon l'une des revendications précédentes, **caractérisé en ce que** la teneur en huile(s) dans le mélange des compositions A, B et C est dans la plage de 10 % à 40 % en poids, plus préférablement de 12 % à 30 % en poids, de préférence de 14 % à 25 % en poids et encore plus préférablement de 14 % à 20 % en poids par rapport au poids total du mélange des compositions A, B et C.
